# EUROPEAN PATENT SPECIFICATION

(11) **EP 2 162 146 B1**
(45) Date of publication and mention of the grant of the patent: **18.12.2013**
(21) Application number: 08759848.8
(22) Date of filing: 21.05.2008
(51) Int. Cl.: A61P 31/04, A61K 38/40

(54) **APOTRANSFERRIN FOR USE IN TREATING BACTERIAL INFECTIONS AS COADJUVANTS IN ANTIBIOTIC THERAPY**
APOTRANSFERRIN ZUR BEHANDLUNG VON BAKTERIELLEN INFEKTIONEN ALS KO-ADJUVANTIEN IN DER ANTIBIOTISCHEN THERAPIE
L' APOTRANSFERRINE POUR TRAITER DES INFECTIONS BACTÉRIENNES EN TANT QUE CO-ADJUVANTS DANS UNE THÉRAPIE PAR ANTIBIOTIQUE

(30) Priority: 21.05.2007 IT LU20070009
(43) Date of publication of application: 17.03.2010
(73) Proprietor: Kedrion S.P.A., 55051 Castelvecchio Pascoli - Barga (IT)
(72) Inventor: SELAN, Laura, I-00139 Roma (IT); ARTINI, Marco, I-00139 Roma (IT); SCOARUGHI, Gian Luca, I-00198 Roma (IT); MARINIELLO, Renato, I-80029 S. Antimo (IT); ASCIONE, Ester, I-80055 Portici (IT); FARINA, Claudio, I-56122 Pisa (IT); MENCONI, Maria Carla, I-55051 Barga (IT)
(74) Representative: Gervasi, Gemma
(86) International application number: PCT/EP2008/056246
(87) International publication number: WO 2008/142102

(56) References cited:
- EP-A- 1 013 283
- WO-A-2005/018701
- WO-A-2006/080970
- US-A- 5 834 424
- US-A- 6 126 955
- SINGH PRADEEP K ET AL: "A component of innate immunity prevents bacterial biofilm development" NATURE, NATURE PUBLISHING GROUP, LONDON, UK, vol. 417, no. 6888, 30 May 2002 (2002-05-30), pages 552-555, XP002453863 ISSN: 0028-0836
- WEINBERG E D: "Suppression of bacterial biofilm formation by iron limitation" MEDICAL HYPOTHESES, EDEN PRESS, PENRITH, US, vol. 63, no. 5, 1 January 2004 (2004-01-01), pages 863-865, XP004602163 ISSN: 0306-9877
- ARDEHALI R ET AL: "The effect of apo-transferrin on bacterial adhesion to biomaterials" ARTIFICIAL ORGANS 2002 US, vol. 26, no. 6, 2002, pages 512-520, XP002509511 ISSN: 0160-564X
- ARDEHALI R ET AL: "The inhibitory activity of serum to prevent bacterial adhesion is mainly due to apo-transferrin" JOURNAL OF BIOMEDICAL MATERIALS RESEARCH - PART A 20030701 US, vol. 66, no. 1, 1 July 2003 (2003-07-01), pages 21-28, XP002509512 ISSN: 0021-9304
- KUROKAWA H ET AL: "Crystal structure of diferric hen ovotransferrin at 2.4 A resolution" JOURNAL OF MOLECULAR BIOLOGY 1995 GB, vol. 254, no. 2, 1995, pages 196-207, XP002509513 ISSN: 0022-2836
- "PDB Apo ovotransferrin"[Online] 29 April 1998 (1998-04-29), XP002509514 Retrieved from the Internet: URL:http://www.ebi.ac.uk/pdbsum/1aiv> [retrieved on 2009-01-08]

## Description

### Field of the invention

The present invention relates to the field of pharmaceutical compositions useful for treating bacterial infections as coadjuvants in antibiotic therapy.

### State of the art

Many bacterial infections follow a chronic pattern and are resistant to common antibiotic therapies due to the capacity of the bacteria sustaining them to adhere to abiotic or biotic substrates; in this way bacteria can assume a sessile phenotype (biofilm) or invade the host's eukaryotic cells. Both strategies render the bacteria resistant to antibiotic therapies.

Acquired knowledge about biofilms has long been attributed to the growth of bacteria in the environment only.

Biofilms were initially observed in the early 1980s on the surfaces of artificial implements introduced into the human body. The clinical manifestations of these infections have for a long time puzzled clinicians and microbiologists due to the scant, non-specific and indolent nature of the symptomatological picture, as well as to their lack of response to treatments administered even in the long term and at high dosages and also, in some cases, to a sudden worsening leading to death. In many of these cases, antibiotic therapy administered at the recommended dosages provides a temporary improvement of symptoms but after suspending the drug, the infection invariably returns with indolent characteristics, until such time as the artificial medical device (prosthesis, pacemaker, catheter, etc.) is removed. It appears from recent observations that the same bacterial biofilms which form on the surfaces of artificial prosthetic devices can also grow on the surfaces of tissues or organs whose integrity is compromised. It follows that the same laws that regulate the preferential growth of bacteria on surfaces are true for all ecosystems, including the human body.

The phenomenon of bacterial resistance to antibiotic therapy has long been known and, until a few years ago, was explained by way of various mechanisms, the main ones being efflux pumps, inactivating enzymes and genetic mutations. These mechanisms do not appear to be responsible for protecting bacteria within biofilms. Susceptibility tests with biofilm models have demonstrated that bacteria survive after antibiotic treatment at concentrations hundreds or even thousands of times greater than the minimum inhibitory concentration effective on the same bacteria maintained suspended in culture medium.

Various human pathologies have so far been described which are related to the presence of biofilms in the human body. Purely for the purposes of classification, a distinction can be made between pathologies connected to the insertion of artificial materials into the body and pathologies connected to biofilm growth on human tissue.

All types of implantable medical devices are potential sites of infection from biofilms, in whatever site in the body they are placed and notwithstanding the type of biomaterial used: vascular prostheses, artificial cardiac valves, cardiac pacemakers, orthopaedic prostheses and endosseous implants, dental implants, central venous catheters, urinary catheters.

The etiological agent responsible for the majority of colonisations belongs to the Staphylococcus species (70-80% of cases) and in particular coagulase-negative staphylococci (90%).

From the time when biofilm adhesion to artificial implantable medical devices was first described, many attempt have been made to prevent infections by biofilms or to eradicate those already initiated. It should be mentioned at this point that, with regard to prosthetic articles, infections have been classified as perioperative (from the first day of implantation to 30 days after), middle distance (one or two years from implantation) and long distance (even after 10 years from the first prosthetic implantation). Perioperative infections can be compared to a normal septic infection at the time of surgery, while the other two are contracted through blood by a transient bacteremia and hence cannot be prevented by antibiotic prophylaxis at the time of surgery. Many attempts have been made to prevent or treat these infections:
- Perioperative antibiotic prophylaxis;
- Long-term antibiotic therapy;
- Use of antibiotic molecules with little steric hindrance, potentially capable of permeating the biofilm matrix;

- Prostheses impregnated with an antibiotic (cardiac valves, urinary catheters, etc.);
- Use of combined antibiotic therapy;
- Prostheses impregnated with anti-biofilm substances (silver salts).

However, none of these attempts has as yet achieved the objective; in this respect all these preventative and therapeutic strategies are effective at controlling biofilm infections without ever succeeding in eradicating them, nor reducing the prevalence of fatal cases.

Normally bacterial colonisation of a mucosa leads to the localized destruction of epithelium and enables an opening to be formed through which the bacteria can reach the highly vascularized sub-mucosa. Some bacteria cross the epithelial mucosa very early on by using specific invasive mechanisms enabling them to penetrate epithelial cells directly despite their not being specialized phagocytes. The capacity of bacteria to invade host cells enables them to utilize various metabolites and nutrients in the cell, to avoid the host's immune response and to resist all antibiotics that do not cross eukaryotic cell membranes (for example cephalosporin). Many chronic/recurring infections are sustained by strains and species capable of adhering to host cells and of invading them; recurring infections are actually caused by bacteremias which re-emerge following cessation of antibiotic therapy cycles (such as urinary infections).

Once anchored to the eukaryotic cell surface, bacteria are in an extremely favourable environment for their multiplication due to the richness of nutrient sources, high water content and precise control of pH, temperature and other physico-chemical parameters. However, the bacteria must also deal with the need to counteract the host's anti-microbial defences and the need to provide themselves with essential elements, which are usually sequestered or not easily accessible, for their metabolic needs.

One of the elements necessary for bacterial growth is iron, contained in high amounts in the human body but not freely usable for the metabolic needs of most pathogenic and non-pathogenic bacteria, as it is mostly found chelated by lactoferrin, transferrin, ferritin and haemin.

Bacteria have developed various mechanisms for capturing iron and each strain can usually employ more than one of them. Many species synthesize specialized molecules (siderophores) which are able to chelate iron very effectively and compete with transferrin and lactoferrin for ion mobilization; some bacteria possess surface receptors that bind transferrin, so as to detach the iron at their surface, and others are able to use haem as a source of iron, whereas yet others can utilize the iron present in hemoglobin or haptoglobin complexes, etc.

On the other hand transferrin is also known to possess antimicrobial activity, to the extent that blood concentrations thereof increase during inflammation and during endotoxic shock.

Human transferrin forms part of a large family of glycoproteins which are important components of the innate immune system and are present in various secretory fluids such as serum and milk. It consists of a single polypeptide chain containing 679 amino-acid residues and two N-glycosidic chains of complex type; the total molecular weight is calculated to be about 80,000 Da with 5.9% being carbohydrate. Under physiological conditions, transferrin saturation with iron is at about 30%. Accordingly, 4 different forms of transferrin coexist in plasma in terms of their iron content, which can be separated and isolated by means of electrophoretic techniques:
1. apotransferrin (apoTf; without ferric ions);
2. monoferric transferrin (iron in the N-terminal domain);
3. monoferric transferrin (iron in the C-terminal domain);
4. diferric transferrin (iron in the two binding sites).

Monoferric or diferric transferrin is also generally known as holotransferrin (holoTf). One of the mechanisms through which transferrin could exert its antimicrobial action comprises direct interaction with bacterial adhesins. From the scientific literature it appears that a variability exists between different Staphylococci strains in the expression of adhesin and surface proteins; these are often expressed in truncated forms (see for example Grundmeier et al. 2004). This leads to the belief that substances which interfere with adhesion, invasion of eukaryotic cells and biofilm formation can have a heterogeneous effect in the various strains.

The antimicrobial activity of transferrin also depends on its capacity to retrieve and bind to iron, its capacity to bind iron being evidently greater in apoTf than in holoTf: as with apolactoferrin (apoLf) (see: Nature 2002 vol. 417 pp. 552-5), apoTf is also a potent iron chelator. It has been demonstrated that apoLf damages Gram-negative bacteria adherent to the outer membrane of eukaryotic cells; in this manner it could prevent cellular invasion in acute infections but induce the release of bacterial lipopolysaccharides into the microenvironment.

From the aforegoing the importance of being able to provide an effective means for countering the aforestated bacterial infections is evident.

### Brief description of the drawings

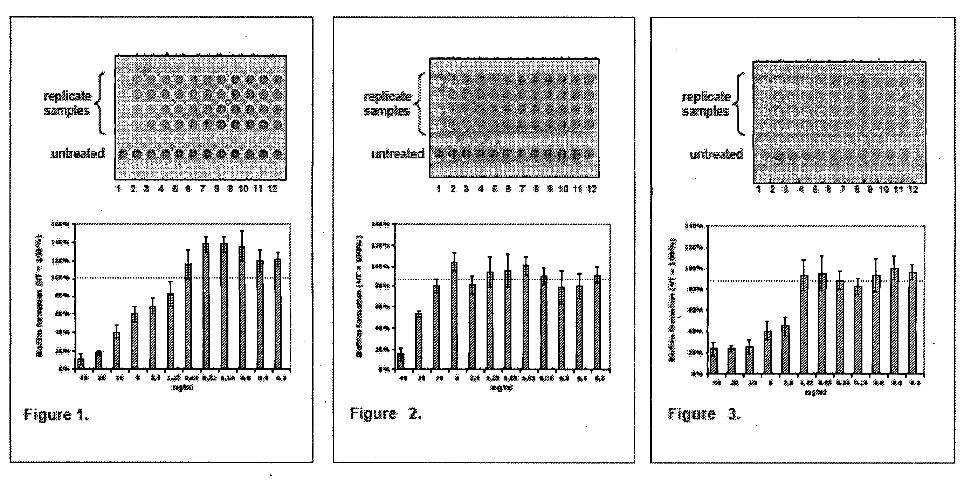

FIGURE 1 describes the Biofilm formation by *S*. *aureus 6538,* in the presence of apoTf Inoculum = 10⁶ cells. apoTf concentration in lanes 1 to 12 diminishes from 40mg/ml to 19.5 µg/ml, as shown by the X axis labels of graphic. Untreated samples: TBS + 10% glucose.
FIGURE 2 describes the Biofilm formation by *S*. *epidermidis O-47*, in the presence of in the presence of apoTf Inoculum = 10⁶ cells. apoTf concentration in lanes 1 to 12 diminishes from 40mg/ml to 19.5 µg/ml, as shown by the X axis labels of graphic. Untreated samples: TBS + 10% glucose.
FIGURE 3 describes the Biofilm formation by *S*. *epidermidis RP62A,* in the presence of in the presence of apoTf Inoculum = 10⁶ cells. apoTf concentration in lanes 1 to 12 diminishes from 40mg/ml to 19.5 µg/ml, as shown by the X axis labels of graphic. Untreated samples: TBS + 10% glucose.

### Detailed description of the invention

It has surprisingly been found, and represents an aspect of the present disclosure that in contrast to indications emerging from previous studies (see for example "The inhibitory activity of serum to prevent bacterial adhesion is mainly due to apotransferrin., J Biomed Mater Res A. 2003 Jul 1;66(1):21-8. Ardehali R, Shi L, Janatova J, Mohammad SF, Burns GL. "The effect of apo-transferrin on bacterial adhesion to biomaterials". Artif Organs. 2002 Jun;26(6):512-20), apoTf is effective as an anti-biofilm molecule in both the early and later stages of its formation.

It is therefore possible to use apoTf for preparing pharmaceutical compositions useful for treating bacterial infections as coadjuvants in antibiotic therapy. Preferably, they are physiological solutions containing apoTf in a quantity comprised between 2 and 40 mg/ml.

### Experimental part

Example: reference and clinical strains of the genus *Staphylococcus* were used to evaluate the effect exerted by apoTf on biofilm production. As stated, the genus *Staphylococcus* is responsible for around 70% of all biofilm sustained human infections of vascular and orthopaedic prostheses.

Techniques have been used for the quantitative evaluation of biofilm formation by using crystal violet stain in the presence and absence of apoTf.

In particular, data relative to the following have been gathered:
- *Staphylococcus aureus* ATCC6538P (reference strain for biofilm production);
- *S. epidermidis* 0-47 (clinical isolate, used as a reference strain for biofilm production: Heilmann *et al.* 1996). This strain was obtained by kind permission of Professor Friedrich Gotz;
- *S. epidermidis* RP62A (ATCC 35984, Christensen et al. 1987).

The experiments aimed at determining a possible interference of apoTf on biofilm formation by Gram-positive bacteria *in vitro* were conducted by reconstituting lyophilized apoTf in sterile water until a stock concentration of 5.8 mg/ml was obtained. The apoTf was then used in graduated concentrations starting from a maximum final concentration of 2.32 mg/ml to a minimum final concentration of 0.9 µg/ml. In the experiments there was no substantial difference between the samples treated with apoTf and the untreated controls.

It was hence decided to increase the maximum final concentration of apoTf up to 40 mg/ml and thereby to include the physiological range of human transferrin concentration in plasma (2.4-3.2 mg/ml).

The experiments on the possible interference of apoTf on biofilm formation *in vitro* were carried out by the following methods:
- formation of biofilms by selected bacterial strains on 96-well polystyrene microplates (Falcon) for 24 hours at 37ºC in TSB growth medium (Oxoid) supplemented with 0.25% glucose.
- so as not to alter the composition of the growth medium for biofilm formation, apoTf was reconstituted directly in the growth medium for biofilm formation, i.e. in TSB (Oxoid)/0.25% glucose. ApoTf was added to the bacterial cultures (concentration of initial inoculum: 10⁸ cells/ml) at the start of incubation, i.e. before biofilm formation, in graduated concentrations, using a 1:2 dilution starting from a maximum concentration of 4 mg per well (40 mg/ml) to a minimum concentration of 1.95 µg per well (19.5 µg/ml).

At the end of the 24 hour incubation time allowed for biofilm formation, the medium and any cells in planktonic phase were gently aspirated from all the wells, then washed twice with 100 µl of sterile PBS x1 solution. The plates were then left to dry at ambient temperature. The bacterial cells adhering to the polystyrene were stained using the method described by C. Heilmann (Heilmann C. et al. 1996) with some modifications: the wells containing biofilm were incubated with a 0.5% crystal violet solution for 1 minute, replacing the 0.1% safranin solution for 30 seconds. Three washes were then carried out in 100 µl of PBS, the multiwells were inverted and left to dry on 3M paper for 15 minutes.

The accompanying figures show the typical results obtained for the three strains used, namely *S. aureus* 6538P, *S. epidermidis* 0-47 and *S. epidermidis* RP62A. Crystal violet penetrates into the extracellular matrix forming the biofilms: to quantify biofilm formation in the wells after drying, the dye was re-suspended with an 80/20 vol/vol ethanol/acetone solution . The amount of crystal-violet present in each well is proportional to the amount of biofilm grown therein, this being quantified by measuring the absorbance of the wells with a ELx800™ Absorbance Microplate Reader (BioTek) at a wavelength of 590 nm. The control used was a well with sterile TSB medium alone, its value being subtracted from the absorbance readings for all samples. Each experimental result derives from the mean readings of four wells, each experiment being conducted in duplicate. The results obtained were translated into the graphs shown in the accompanying figures.

The analysis of the effects of apoTf on biofilms of Staphylococci strains has clearly shown that the molecule possesses a biological activity on the biofilms of all the Staphylococcal strains analyzed within the concentration range used.

Therefore apoTf is an effective molecule for reducing adhesion and invasion of pathogenic bacteria in cultured human cells.

### Experimental part

Example: we have found that invasion of eukaryotic cells by *S*. *aureus* 6538P was influenced by apoTf concentrations of between 100 and 400 µg/ml. Specifically, the invasive capacity of the strain under study was reduced (30% - 50%) by the presence of apoTf at concentrations ≥ 200 µg/ml. Treatment with apoTf did not modify the number of bacteria adherent to eukaryotic cells, but reduced the number of intracellular bacteria and reduced invasiveness by up to 50%.

With this objective, we used adherent epithelial cells of human adenocarcinoma (HeLa). In order not to alter the composition of the cell growth medium, the apoTf was reconstituted in PBS to a stock concentration of 100 mg/ml. In a second group of experiments we used a single final apoTf concentration comparable with its concentration in plasma and equal to 5 mg/ml (25 µl in 0.5 ml of cell culture). The apoTf was added to the cell cultures after replacing the complete medium with serum- and antibiotic-free medium 10 minutes before adding the bacteria for infection. This latter was carried out 24 hours after transferring the HeLa cells into 24-well multiwell plates.

The inoculum was equal to 10⁵ bacteria per 55,000 eukaryotic cells/well. To assay the invasive capacity of the bacteria, the bacteria that survived a treatment with an antibiotic effective on the strain used but non-effective on eukaryotic cells (dose/MIC-MBC) were counted: after one hour of incubation at 37 ºC under a 5% CO₂ atmosphere, 50 µg/ml of gentamicin were added and the cells incubated for 1 hour. The lysate of eukaryotic cells containing the intracellular bacteria surviving the antibiotic treatment was used for counting the bacteria that had invaded the cells themselves.

To assay the adhesive capacity of the bacterial strain analyzed, a second series of experiments without antibiotic treatment was carried out. The number of adherent bacteria was obtained by subtracting the number of bacteria that had invaded the cells from the total bacterial count (the sum of adhered bacteria and bacteria having invaded eukaryotic cells). To avoid the bacterial count also including bacterial cells not perfectly adhering or dispersed in the cell culture medium, the cells plus bacteria mixture was washed twice with PBS 1X.

For both experimental sets the cells were lysed with H₂O + 0.025% Triton X140.

After centrifugation, the lysate was re-suspended in 100 µl of PBS 1X and plated onto solid TSA medium to enable the bacterial colonies to be counted (18 hours' incubation at 37ºC). Each individual condition was carried out in triplicate to achieve statistical confirmation of the results.

In these experiments also, despite the apoTf concentration being one order of magnitude greater than in the preceding experiment, reduction of invasiveness was found to be around 50%.

Both the experiments demonstrated a reduction in bacterial adhesion of between 30 and 50% in treated compared to non-treated bacteria.

ApoTf exerts bactericidal or bacteriostatic effects. It can therefore be used as a synergist to bactericidal or bacteriostatic antibiotic action and optionally alone in preparations for topical use.

### Experimental part

Example: the MIC (Minimum Inhibitory Concentration) and MBC (Minimal Bactericidal Concentration) of apoTf and holoTf were evaluated. In this stage of the experiment *S*. *aureus* 6538P was used. The transferrins in our possession were re-suspended directly in BHI so as not to change the composition of the medium used in the various experiments.

The MICs were carried out in bacteriology tubes containing 1 ml of BHI medium (Brain Heart Infusion) with graduated concentrations of apoTf and holoTf starting from a maximum of 20 mg/ml, to a minimum of 39 µg/ml. BHI medium was used in place of standard MIC medium, namely MHB (Mueller Hinton Broth), as it is the medium used for extraction of surface proteins. In all the tubes, except the negative control, an inoculum was introduced equal to 5 x 10⁶ cultured bacteria in exponential growth; the tubes were allowed to grow at 37ºC in a wheel. The evaluation was carried out on the following day by determining the turbidity of the cultures. The cultures that were less turbid than the transferrin-free positive control were used for a viability count on the plates (evaluation of CFUs, colony forming units) in order to verify the bactericidal capacity of the compound at a given concentration (MBC).
Table 1 shows the MIC results.

**TABLE 1**

| mg/ml | ApoTF | HoloTf |
|---|---|---|
| 20 | - | + |
| 10 | +- | + |
| 5 | +- | + |
| 2.5 | + | + |
| 1.25 | + | + |
| 0.625 | + | + |
| 0.312 | + | + |
| 0.156 | + | + |
| 0.078 | + | + |
| 0.039 | + | + |

| | | |
|---|---|---|
| The MIC concentrations are the following: MIC apoTf between 10 and 20 mg/ml MIC holoTf>20 mg/ml | | |

Table 2 shows the results of MBC expressed as CFU/ml

**TABLE 2**

| mg/ml | ApoTF | Holo Tf |
|---|---|---|
| 20 | < 10 | 9.45 × 10⁷ |
| 10 | 1.26 × 10⁷ | 1.97 × 10⁸ |
| 5 | 1.90 × 10⁷ | 2.00 × 10⁸ |
| 0.039 | 1.27 × 10⁸ | 1.79 × 10⁸ |
| Pos. c. | 2.00 × 10⁸ | 2.00 × 10⁸ |

| | | |
|---|---|---|
| The MBC concentrations are the following: MBC apoTf between 10 and 20 mg/ml MBC holoTf >20 mg/ml | | |

It can therefore be stated that in the case of the transferrin molecules used in *S*. *aureus* 6538P, the MIC corresponds to the MBC.

## Claims

1. Pharmaceutical physiological solutions comprising apotransferrin in a concentration comprised between 4 and 30 mg/ml for use in the treatment of bacterial infections as coadjuvants in antibiotic therapy.

2. Pharmaceutical compositions according to claim 1 further comprising an antibiotic agent for use in the treatment of bacterial infections.

3. Pharmaceutical compositions for use according to any of claims 1-2 for use in reducing adhesion and/or invasion of pathogenic bacteria.

## Patentansprüche

1. Pharmazeutische physiologische Lösungen, umfassend Apotransferrin in einer Konzentration, die zwischen 4 und 30 mg/ml liegt, zur Verwendung bei der Behandlung von bakteriellen Infektionen als Coadjuvantien in der antibiotischen Therapie.

2. Pharmazeutische Zusammensetzungen nach Anspruch 1, die darüber hinaus ein antibiotisches Mittel umfassen, zur Verwendung bei der Behandlung von bakteriellen Infektionen.

3. Pharmazeutische Zusammensetzungen zur Verwendung nach einem der Ansprüche 1 bis 2 zur Verwendung beim Verringern einer Adhäsion und/oder Invasion von pathogenen Bakterien.

## Revendications

1. Solutions physiologiques pharmaceutiques comprenant de l'apotransferrine en une concentration comprise entre 4 et 30 mg/mL pour leur utilisation dans le traitement d'infections bactériennes en tant que coadjuvants dans une thérapie antibiotique.

2. Compositions pharmaceutiques selon la revendication 1, comprenant en outre un agent antibiotique, pour leur utilisation dans le traitement d'infections bactériennes.

3. Compositions pharmaceutiques pour leur utilisation selon l'une quelconque des revendications 1 à 2, pour réduire l'adhésion et/ou l'invasion de bactéries pathogènes.
